# EUROPÄISCHE PATENTSCHRIFT

(11) **EP 0 772 558 B1**
(45) Veröffentlichungstag und Bekanntmachung des Hinweises auf die Patenterteilung: **09.12.1998**
(21) Anmeldenummer: 95912248.2
(22) Anmeldetag: 16.03.1995
(51) Int. Cl.: B65F 1/16, B65F 1/14, B65D 45/30, B65D 21/02, B65F 1/06

(54) **STERILISATIONSBEHÄLTER, INSBESONDERE FÜR KRANKENHAUSABFÄLLE**
STERILISATION CONTAINER, IN PARTICULAR FOR HOSPITAL WASTE
RECIPIENT DE STERILISATION, NOTAMMENT POUR DECHETS D'HOPITAUX

(30) Priorität: 16.03.1994 DE 9404372 U; 16.03.1994 DE 9404822 U
(43) Veröffentlichungstag der Anmeldung: 14.05.1997
(73) Patentinhaber: STERIFANT INTERNATIONAL HOLDING AG, 1466 Luxembourg (LU)
(72) Erfinder: KAULEN, Ernst, D-66265 Heusweiler (DE); HEGGER, Roland, D-66265 Heusweiler (DE)
(74) Vertreter: Berkenfeld, Helmut, Dipl.-Ing.
(86) Internationale Anmeldenummer: EP9500981
(87) Internationale Veröffentlichungsnummer: WO9525054

(56) Entgegenhaltungen:
- EP-A- 0 455 172
- AU-D- 826 366
- DE-U- 9 207 227
- US-A- 4 082 184
- US-A- 4 678 216
- US-A- 4 819 795

## Beschreibung

Die Erfindung betrifft einen Sterilisationsbehälter, insbesondere für Krankenhausabfälle, mit einem Kübel und einem auf diesen aufsetzbaren Deckel aus einem zähen, für Mikrowellen durchlässigen und auch über die Siedetemperatur von Wasser hinaus dehnungsfesten Kunststoff und mit einer Abdichtung zwischen dem oberen Rand des Kübels und dem aufgelegten Deckel.

Dieser Sterilisationsbehälter dient in erster Linie zum Aufnehmen, Lagern und Entsorgen von Krankenhausabfällen, das heißt infektiösen Abfällen. Er kann aber auch in human- und veterinärmedizinischen, biologischen und gentechnischen Laboratorien, in Hotels und Gaststätten und in Kantinen angewendet werden.

Zum Sterilisieren von infektiösen, auf andere Weise kontaminierten oder die Gesundheit gefährdenden Abfällen werden diese erhitzt, überlicherweise mit Dampf. Hierzu muß eine ausreichende Menge ausreichend heißen Dampfes durch die Abfälle durchgeleitet werden. Bei Durchleiten von heißem Dampf durch die Abfälle besteht die Gefahr, daß kontaminierte Luft und kontaminierter Dampf in die Umgebung entweichen. Um dies zu vermeiden, muß der heiße Dampf in den Abfällen selbst bzw. im Sterilisationsbehälter erzeugt werden. Hierzu können Mikrowellen verwendet werden. Die Verwendung von Mikrowellen bedingt jedoch einen ausreichend hohen Flüssigkeitsanteil der Abfälle. Trockene oder nicht ausreichende feuchte Abfälle lassen sich mit Mikrowellen nicht erhitzen und damit nicht sterilisieren. Solche Abfälle werden vor dem Erhitzen durch Zugabe von Wasser angefeuchtet. Bei einem Erhitzen dieser Abfälle bildet sich Dampf. Damit steigt der Druck im Sterilisationsbehälter. Dies führt zu einem Anstieg der Verdampfungstemperatur. Es kommt zu einem Gleichgewicht zwischen dem Dampfdruck und dem Druck im Behälter. Die sogenannte Sattdampfatmosphäre stellt sich ein. Diese ermöglicht eine optimale Sterilisation. Bei Erreichen der Sattdampfatmosphäre steigt die Temperatur nicht weiter an. Der gasförmige Dampf wird durch die Mikrowellen nicht weiter erhitzt.

Ein Sterilisationsbehälter der eingangs genannten Gattung aus einem für Mikrowellen durchlässigen Kunststoff ist bekannt (DE 3 833 281 C1). Weiter ist ein Verfahren bekannt, mit dem solche mit Krankenhausabfällen gefüllte Sterilisationsbehälter durch die Einwirkung von Mikrowellen erwärmt werden können (EP 410 306 A2). Bei diesem Verfahren wird eine Injektionsnadel in den Deckel des Sterilisationsbehälters eingestochen. Wasser wird durch die Injektionsnadel zugegeben. Anschließend werden die Sterilisationsbehälter durch die Einwirkung von Mikrowellen erhitzt. Damit werden die in diesem enthaltenen Krankenhaus- oder sonstigen Abfälle sterilisiert. Sie werden vom Sondermüll zum Normalmüll und können auf normale Weise entsorgt werden. Es hat sich gezeigt, daß die mit dem beschriebenen Sterilisieren verbundenen Kosten weiter unter den Kosten liegen, die durch das Entsorgen der Krankenhausabfälle als Sondermüll entstehen. Deshalb sind Krankenhäuser und andere Einrichtungen, in denen infektiöser Sondermüll anfällt, bestrebt, ihren Sondermüll auf die beschriebene Weise in normalen Müll umzuwandeln. Voraussetzung hierfür ist ein Müll- oder Sterilisationsbehälter, in dem die Abfälle wie übliche Abfälle eingegeben und gesammelt werden können. Weiter muß sichergestellt werden, daß ein gefüllter Sterilisationsbehälter nicht unabsichtlich oder absichtlich oder mutwillig geöffnet und die infektiösen Abfälle damit freigegeben werden. Schließlich muß der Sterilisationsbehälter auch so ausgebildet sein, daß er ohne weiteres Zutun mit dem vorstehend angedeuteten Verfahren erhitzt und sein Inhalt damit sterilisiert werden kann.

Von diesem Stand der Technik ausgehend, liegt der Erfindung die Aufgabe zugrunde, den bekannten Sterilisationsbehälter so auszubilden, daß er im praktischen Betrieb eines Krankenhauses zur Aufnahme, zum Sammeln, zum Sterilisieren und schließlich zum Entsorgen von Abfällen eingesetzt werden kann. Die Lösung für diese Aufgabe ergibt sich nach der Erfindung bei einem Sterilisationsbehälter der eingangs genannten Gattung mit einer abnehmbaren Verschlußanordnung zum Halten des Deckels auf dem Kübel sowohl bei der Lagerung eines gefüllten Sterilisationsbehälters als auch entgegen dem Druck des in ihm beim Erhitzen zur Sterilisation erzeugten Dampfes. Die Verschlußanordnung hält den Deckel auch schon nach dem Befüllen des Kübels auf diesem fest, so daß die Gefahr eines Öffnens und Austretens der Abfälle aus dem Kübel vermieden wird. Damit können die gefüllten Sterilisationsbehälter auch so lange sorglos gelagert und gesammelt werden, bis eine für ein wirtschaftliches Erhitzen und Sterilisieren erforderliche Anzahl angefallen ist.

Neue und auch nach einem Sterilisieren geleerte Kübel sollen raumsparend gelagert werden können. Hierzu sieht die Erfindung vor, daß der Kübel aus mehreren sich in ihrem Durchmesser von oben nach unten verjüngenden Abschnitten besteht. Dadurch werden die Kübel stapelbar. Die zwischen den einzelnen Abschnitten entstehenden treppenförmigen Absätze erhöhen auch die Festigkeit eines Kübels.

Eine weitere zweckmäßige Ausgestaltung ist gekennzeichnet durch in Umfangsrichtung verteilt angeordnete, von der Innenseite des Bodens eines Kübels an dessen Innenwand nach oben verlaufende und in ihrer radialen Stärke abnehmende Rippen zur Auflage eines in diesen Kübel eingesetzten weiteren Kübels. Auch diese Rippen erhöhen die Festigkeit des Kübels. Weiter verhindern sie, daß ein zum Stapeln in einen Kübel eingesetzter weiterer Kübel sich so fest an die Innenwand des ersteren anlegt, daß er nur noch mit erhöhtem Kraftaufwand aus diesem herausgezogen werden kann. Eine Weiterbildung dieser Ausführungsform ist gekennzeichnet durch auf der Außenwand eines Kübels in einem Abstand unter dessen oberem Rand in Umfangsrichtung verteilt angeordnete radiale VorSprünge, wobei der Abstand zum oberen Rand kleiner als die Höhe der Rippen ist. Diese Vorsprünge bilden Handgriffe zum Handhaben des Sterilisationsbehälters. Weiter bilden sie eine Auflage für die Verschlußanordnung bei deren Nichtgebrauch.

Beim Erhitzen des Sterilisationsbehälters steigt der Druck in diesem an. Zum Vermeiden eines Auswölbens des Bodens durch diesen Druck ist in einer zweckmäßigen Ausgestaltung vorgesehen, daß der Boden des Kübels nach oben gewölbt ist.

Eine weitere Ausgestaltung ist gekennzeichnet durch einen auf der Unterseite des Bodens eines Kübels mittig angeordneten Rohrstutzen, dessen Höhe unter der Auswölbung des Bodens liegt. Auch dieser Rohrstutzen erhöht die Festigkeit des Bodens. Weiter dient er zum Zentrieren, wenn mit dem Deckel verschlossene Sterilisationsbehälter übereinandergestapelt werden. Zum Erhöhen der Festigkeit des Bodens dienen auch in Umfangsrichtung verteilt vom Umfang des Rohrstutzens zur Unterseite des Bodens eines Kübels verlaufende und in ihrer Höhe abnehmende Rippen.

Es wurde ausgeführt, daß der Boden nach oben gewölbt ist, um dem beim Erhitzen entstehenden höheren Innendruck standzuhalten. Zum gleichen Zweck ist der Deckel nach unten gewölbt.

Zum weiteren Erhöhen der Festigkeit des Deckels und zum Zentrieren übereinandergestapelter Sterilisationsbehälter dient ein auf der Oberseite des Deckels mittig angeordneter Rohrstutzen.

Eine weitere zweckmäßige Ausgestaltung ist gekennzeichnet durch einen auf der Innenseite des Deckels nahe an dessen Umfang umlaufenden und bei ineinandergesetzten Kübeln an der Innenwand eines Kübels anliegenden inneren Ringsteg. Dieser Ringsteg zentriert einen Deckel beim Aufsetzen auf den Kübel.

Zum weiteren Erhöhen der Festigkeit des Deckels dient eine Ausführungsform, die gekennzeichnet ist durch in Umfangsrichtung verteilt vom Rohrstutzen auf der Oberseite des Deckels zu dessen Umfang verlaufende und in ihrer Höhe abnehmende Rippen. Zum gleichen Zweck sind in Umfangsrichtung verteilt vom Umfang des Deckels auf dessen Unterseite zur Mitte hin verlaufende und in ihrer Höhe abnehmende Rippen vorgesehen.

Es wurde ausgeführt, daß vor dem Erhitzen und Sterilisieren eine Injektionsnadel in den Deckel eingestochen wird. Diese muß den Deckel durchdringen. Um dies zu erleichtern, ist im Rohrstutzen des Deckels eine mit diesem über eine Ringkerbe verbundene herausdrückbare Scheibe angeordnet. Eine alternative Ausführungsform ist gekennzeichnet durch ein im Rohrstutzen des Deckels angeordnetes konisch verlaufendes Rohrstück mit von oben nach unten abnehmendem Durchmesser und einem in dieses hineindrückbaren Stopfen aus einem elastischen Werkstoff. Zweckmäßig ist der Stopfen hohl. Eine zweckmäßige Weiterbildung des Stopfens ist gekennzeichnet durch in dessen Mantel vorgesehene axial verlaufende Schlitze und einen am unteren Ende vorgesehenen, das Rohrstück untergreifenden Flansch. Die Schlitze erleichtern das Zusammendrücken des Stopfens beim Hineinschieben in das konisch verlaufende Rohrstück. Der dieses untergreifende Flansch hält den Stopfen sicher in diesem Rohrstück. Ein O-Ring ist auf dem Mantel des Stopfens gehalten. Er erhöht die Dichtigkeit zwischen Stopfen und Rohrstück. Bei einem mit infektiösem Krankenhausmüll gefüllten Sterilisationsbehälter muß der Deckel vollständig dicht auf dem Kübel aufsitzen. In einer zweckmäßigen Ausgestaltung ist hierzu vorgesehen, daß ein Umfangsflansch am oberen Rand des Kübels und ein dazu passender Umfangsflansch am Rand des Deckels vorgesehen ist, eine Nut in diesen ausgebildet und ein 0-Ring in dieser Nut aufgenommen ist. Dabei sollten die Abmessungen der Nut so auf die Abmessungen des O-Ringes abgestimmt sein, daß dieser im Betriebszustand zusammengepreßt ist.

Die Verschlußanordnung ist ein wesentliches Element des erfindungsgemäßen Sterilisationsbehälters. Sie muß zum Anklemmen des Deckels an den Kübel leicht an diese angesetzt werden können. Weiter muß sie bei Nichtgebrauch an einer Stelle untergebracht werden können, wo sie zum Gebrauch sofort zur Verfügung steht. Weiter muß sie so ausgebildet sein, daß sie sich nur mit Mühe vom Sterilisationsbehälter lösen läßt. Schließlich muß dafür gesorgt sein, daß ihr bösartiges Abnehmen sofort erkannt wird. In einer zweckmäßigen Ausgestaltung ist hierzu vorgesehen, daß die Verschlußanordnung ein Spannring ist und dieser auf die Umfangsflansche von Kübel und Deckel auflegbar ist. Die Erfindung schlägt weiter vor, daß der Spannhebel des Spannringes in Schließstellung durch einen in ihn und den Spannring einschiebbaren Sicherungsstift verriegelbar ist. Dieser Sicherungsstift hält den Spannring sicher am Sterilisationsbehälter. Ein Öffnen des Spannringes verlangt das Herausnehmen des Sicherungsstiftes. Dieser ist so ausgebildet, daß dies sofort erkennbar wird.

In einer zweckmäßigen Ausgestaltung ist vorgesehen, daß der Spannring und der Spannhebel in Schließstellung übereinanderliegende Öffnungen aufweisen und der Sicherungsstift in diese einschiebbar ist. Zweckmäßig sind die Öffnungen Längsschlitze. Diese Längsschlitze sichern eine gute Führung und einen guten Halt des Sicherungsstiftes. Eine radial vorspringende Lasche ist noch an den Spannring angesetzt. Dessen Längsschlitz ist in dieser Lasche ausgebildet.

Eine weitere zweckmäßige Ausgestaltung zeichnet sich dadurch aus, daß der Sicherungsstift zwei an ihren oberen Enden durch einen Steg verbundene Schenkel aufweist und ein Handgriff über eine Sollbruchstelle mit dem Steg verbunden ist. Zweckmäßig hat der Handgriff die Form eines Ringes. Weiter weisen die beiden Schenkel eine Sicherung auf, die mit einer Begrenzung eines Längsschlitzes verrastet. Diese Sicherung besteht zweckmäßig aus sich von beiden Seiten an die Begrenzung des Längsschlitzes anlegenden Vorsprüngen der Schenkel. Diese sind an den freien unteren Enden der Schenkel angeordnet. Diese sind elastisch zusammendrückbar. Infolge dieser Elastizität der Schenkel läßt sich der Sicherungsstift ohne besonderen Kraftaufwand in die Längsschlitze einschieben. Damit wird der Spannhebel mit dem Spannring verriegelt. Zu dessen Öffnen muß der Sicherungsstift aus den Längsschlitzen herausgezogen werden. Der befugte Anwender verwendet hierzu ein besonderes Werkzeug. Mit diesem werden die unteren freien Enden der beiden Schenkel zusammengedrückt. Durch Ziehen am Griff kann der Sicherungsstift dann aus den Längsschlitzen herausgezogen werden. Ein Unbefugter wird den Griff erfassen und versuchen, den Sicherungsstift mit diesem herauszuziehen. Dies führt jedoch zu einem Bruch der Sollbruchstelle. Der Griff reißt ab. Der größere Teil des Sicherungsstiftes verbleibt im Spannring und hält den Spannhebel in Schließstellung.

Es hat sich eingebürgert, sogenannte Müllbeutel in Müllbehälter einzulegen. Sie nehmen den Abfall auf und verhindern eine Beschmutzung der Innenwand des Müllbehälters. Damit entfallen eine größere Reinigung und Desinfektion des Müllbehälters. Diesem Zweck dient ein in den Kübel einlegbarer Innenbeutel aus einem in seinem oberen Bereich elastisch verformbaren Kunststoff. Dieser Bereich wird um den oberen Rand des Kübels umgelegt. Die Elastizität erleichtert dieses Umlegen. Zweckmäßig ist der obere Bereich etwa doppelt so lang wie der um den oberen Rand des Kübels umgeschlagene Teil des Innenbeutels. Nur dieser obere Bereich ist elastisch ausgebildet. Der größere, im Kübel befindliche Bereich des Innenbeutels ist aus einem festeren und nichtelastischen Werkstoff. Bei einer Elastizität des Innenbeutels auch in diesem Bereich würde er durch schwere oder scharfkantige Abfälle ausbeulen oder sogar aufreißen. Zur Vereinfachung der Herstellung und zum Senken der Kosten des Innenbeutels weist der verformbare obere Bereich eine geringere Stärke als der übrige, nicht elastisch verformbare Bereich des Innenbeutels auf. Zweckmäßig sind die beiden Bereiche des Innenbeutels miteinander verschweißt.

Zum Erhöhen der Festigkeit des Kübels sind an dessen Außenwand noch bis zu den Unterseiten der Vorsprünge verlaufende Rippen vorgesehen.

Der Deckel muß zentriert, fest und sicher auf dem Kübel aufsitzen. Hierzu ist in einer weiteren Ausgestaltung vorgesehen, daß auf der Innenseite des Deckels an dessen Umfang ein äußerer Ringsteg verläuft, dieser mit dem inneren Ringsteg einen Ringspalt einschließt und der obere Rand eines Kübels bei ineinandergesetzten Kübeln im Ringspalt aufgenommen ist. Damit der obere Rand des Kübels leicht in diesen Ringspalt hineingleitet und sich dabei zentriert, verjüngt sich der Ringspalt nach oben und endet in einer Ausrundung.

Am Beispiel der in der Zeichnung gezeigten Ausführungsformen wird die Erfindung nun weiter beschrieben. In der Zeichnung ist:
- Fig. 1: eine Seitenansicht des erfindungsgemäßen Sterilisationsbehälters, wobei das Ineinanderstapeln von zwei Sterilisationsbehältern in strichpunktierten Linien angedeutet ist,
- Fig. 2: eine auseinandergezogene Darstellung des Kübels, des Deckels, des Spannringes und des Innenbeutels,
- Fig. 3: eine Aufsicht auf den Deckel,
- Fig. 4: ein Querschnitt durch einen Deckel mit einem in strichpunktierten Linien angedeuteten, auf diesen Deckel aufgesetzten Kübel,
- Fig. 5: eine Sicht in einen Kübel mit der Aufsicht auf den Boden,
- Fig. 6: eine Aufsicht auf den Spannring,
- Fig. 7: in vergrößertem Maßstab eine Teil-Seitenansicht, teilweise im Schnitt, der mit dem Sicherungsstift zusammenwirkenden Teile des Spannringes bei Beginn des Einschiebens des Sicherungsstiftes,
- Fig. 8: eine Ansicht ähnlich Fig. 7 bei vollständig eingeschobenem Sicherungsstift und beim Versuch, diesen mit dem Griff herauszuziehen,
- Fig. 9: in vergrößertem Maßstab ein Teil-Längsschnitt durch die miteinander zusammenwirkenden Teile von Kübel und Deckel und Spannring bei noch geöffnetem Sterilisationsbehälter,
- Fig. 10: ein Teil-Längsschnitt ähnlich Fig. 9 bei geschlossenem Sterilisationsbehälter,
- Fig. 11: in vergrößertem Maßstab ein Teil-Längsschnitt durch einen geöffneten Kübel und den Spannring bei Nichtgebrauch,
- Fig. 12: ein Teil-Längsschnitt ähnlich Fig. 11 mit eingelegtem Innenbeutel,
- Fig. 13: in vergrößertem Maßstab ein Teil-Längsschnitt des in Fig. 4 in dem Kreis XIII befindlichen Gebietes bei Verwendung des Stopfens und der angepaßten Ausführungsform des Deckels vor dem Einschieben des Stopfens und
- Fig. 14: ein Teil-Längsschnitt ähnlich Fig. 13 bei eingeschobenem Stopfen.

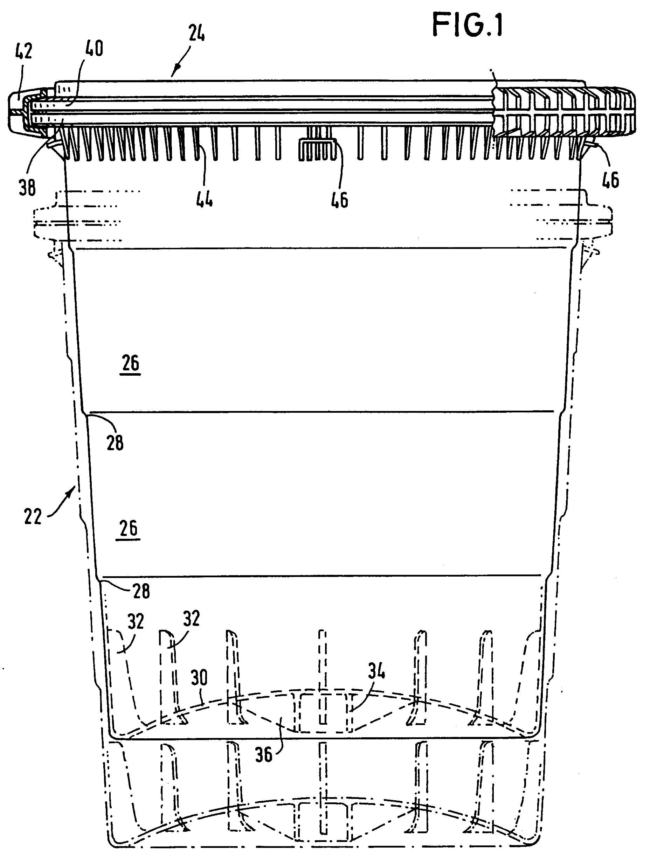

Fig. 1 zeigt den Kübel 22 mit einem Deckel 24. Der Kübel 22 weist mehrere sich von oben nach unten verjüngende Abschnitte 26 auf. An Absätzen 28 stoßen sie aufeinander. Der Boden des Kübels 22 ist mit 30 bezeichnet. Er ist nach innen oder oben gewölbt. In Umfangsrichtung verteilt sind unter gegen-seitigem Abstand Rippen 32 in dem unteren Abschnitt 26 und auf dem Boden 30 angeordnet. Sie weisen eine von unten nach oben abnehmende radiale Stärke auf. Ein Rohrstutzen 34 ist mittig auf der Unterseite des Bodens 30 angeordnet. Von ihm verlaufen in Umfangsrichtung verteilt radial Rippen 36 zum Boden 30. Der Kübel weist an seinem oberen Rand noch einen Umfangsflansch 38 auf. Der Deckel 24 liegt mit einem Umfangsflansch 40 auf diesem auf. Ein Spannring 42 umschließt die beiden Umfangsflansche 38 und 40 und hält den Deckel 24 auf dem Kübel 22. In Umfangsrichtung verteilt sind unter gegenseitigen Abständen Rippen 44 am oberen Ende des Kübels 22 angeordnet. Sie verlaufen von dessen Außenwand bis unter den Umfangsflansch 38. In Umfangsrichtung verteilt sind weiter vier radiale Vorsprünge 46 auf der Außenwand des Kübels 26 angeordnet. Sie befinden sich in einem kurzen Abstand unter dem Umfangsflansch 38. In strichpunktierten Linien ist ein weiterer Sterilisationsbehälter angedeutet. Auf seinen Rippen 32 steht der eben beschriebene, in voll ausgezogenen Linien dargestellte Sterilisationsbehälter auf.

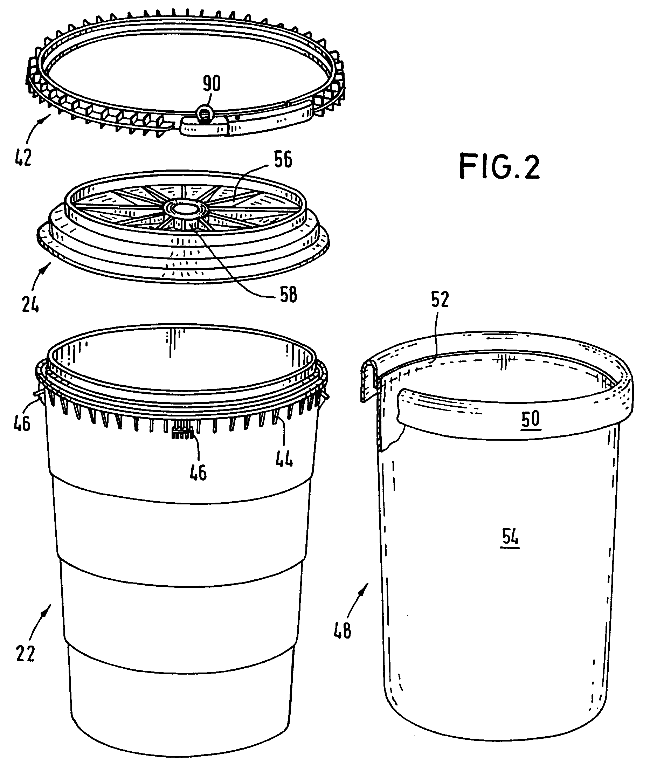

Fig. 2 zeigt zusätzlich den Innenbeutel 48 mit einem oberen Bereich 50, einer Überlappung 52 und dem unteren Bereich 54. Entlang der Überlappung 52 sind die beiden Bereiche 50 und 54 miteinander verschweißt. Der Innenbeutel 58 besteht aus Kunststoffolien. Die den oberen Bereich 50 bildende Folie ist dünner und damit elastischer als die den unteren Bereich 54 bildende Folie. Der Deckel 24 weist auf seiner Oberseite Rippen 56 und mittig einen Rohrstutzen 58 auf. Zum Gebrauch wird der Innenbeutel 54 in den Kübel 22 eingeschoben. Der obere Bereich 50 umschließt dabei den Umfangsflansch 38. Er kann länger als gemäß der Darstellung in Fig. 2 sein.

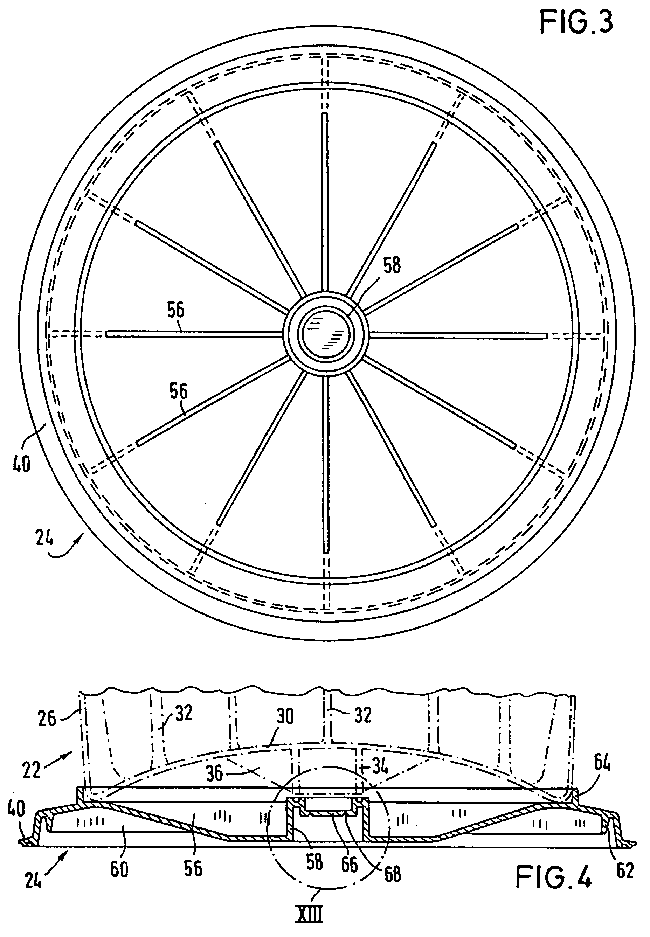

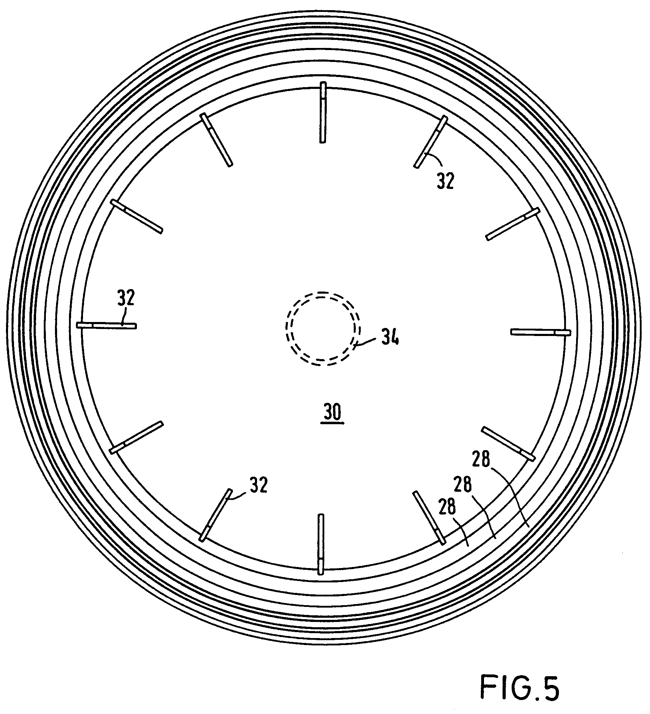

Fig. 3 zeigt die auf der Oberseite des Deckels 24 zwischen dem Rohrstutzen 58 und dem Umfang verlaufenden Rippen 56. Fig. 4 zeigt die Hochwölbung des Deckels 24. Die Rippen 56 auf seiner Oberseite verlaufen vom mittig angeordneten Rohrstutzen 58 radial nach außen bis zu der Stelle der höchsten Hochwölbung des Deckels 24. Auf seiner Unterseite sind in Um-fangsrichtung verteilt weitere radial verlaufende Rippen 60 vorgesehen. An ihren inneren Enden sind sie komplementär zu den Rippen 56 ausgebildet. Gemeinsam erhöhen beide Rippen 56 und 60 die Festigkeit des Deckels 24. Der Deckel 24 weist weiter noch einen unteren Ringsteg 62 und einen oberen Ringsteg 64 auf. Fig. 4 zeigt in strichpunktierten Linien einen auf den Deckel 24 aufgesetzten Kübel 22 eines weiteren Sterilisationsbehälters. Er liegt mit dem Rand seines Bodens 30 am Ringsteg 64 an. Durch diesen wird er zentriert und gehalten. Fig. 4 zeigt weiter eine einstückig mit dem Rohrstutzen 58 ausgebildete Scheibe 66 mit einer in dieser vorgesehenen Ringkerbe 68. Diese dient zum Hineinstoßen einer Injektionsnadel zum Zuführen von Wasser. Fig. 5 zeigt den Boden 30 von oben mit dessen bereits beschriebenen Absätzen 28, den Rippen 32 und dem Rohrstutzen 34.

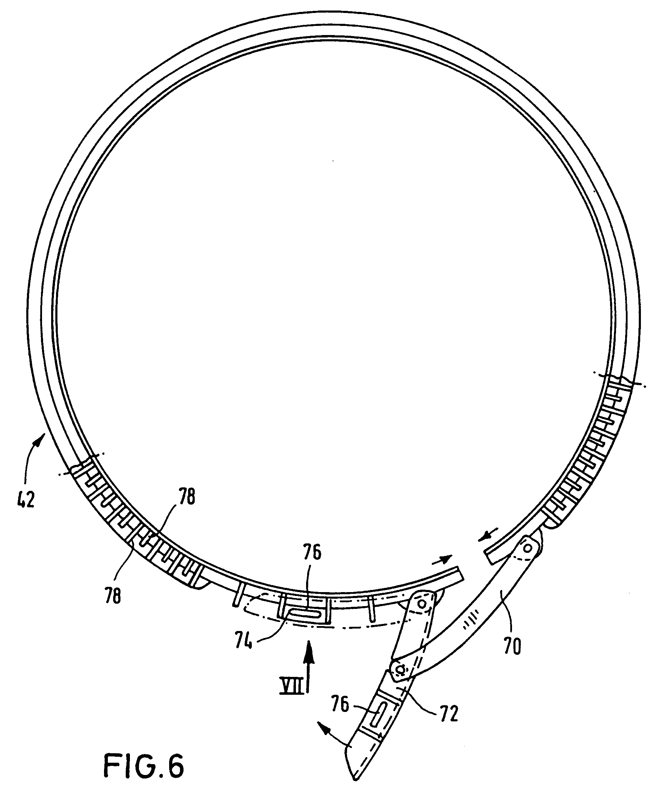

Fig. 6 zeigt den Spannring 42 in größerer Ausführlichkeit. Er weist ein Verbindungselement 70 zwischen seinen beiden Enden und einen Spannhebel 72 auf. In dessen Schwenkbereich sitzt eine Lasche 74 auf dem Spannring 42. In dieser und im Spannhebel 72 sind Längsschlitze 76 vorgesehen. Bei geschlossenem Spannring 42 liegen diese übereinander. Radialstege 78 sind unter gegenseitigem Abstand in Umfangsrichtung verteilt auf dem Spannring 42 angeordnet.

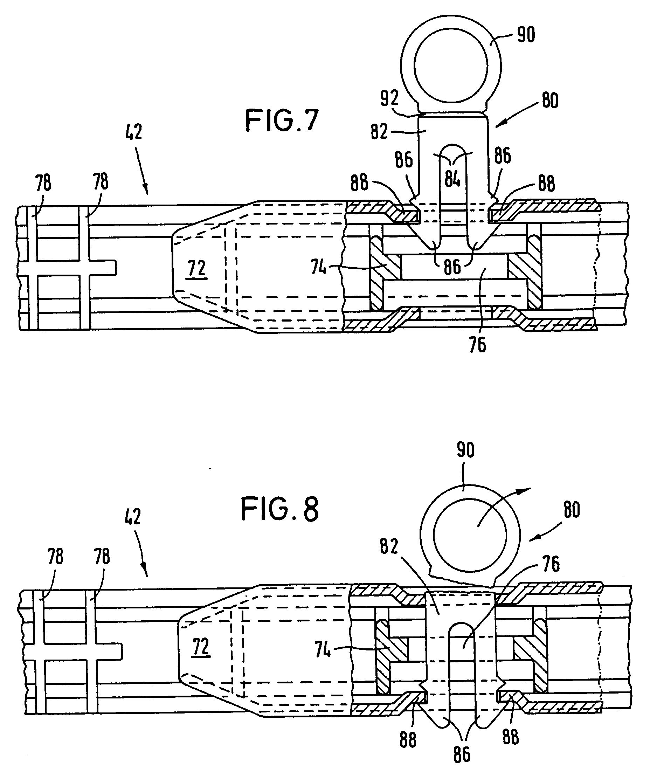

Die Figuren 7 und 8 zeigen einen Teil eines geschlossenen Spannringes 42 in Blickrichtung des in Fig. 6 eingezeichneten Pfeiles VII. Die Figuren 7 und 8 zeigen den Sicherungsstift 80 mit dem Steg 82, den beiden von diesem ausgehenden Schenkeln 84 und den Vorsprüngen 86 an deren unteren Enden. Sie umfassen Flansche 88 des Spannhebels 72. Ein Handgriff 90 ist über eine Sollbruchstelle 92 mit dem Steg 82 verbunden. Fig. 7 zeigt den Sicherungsstift 80 zu Beginn des Einschiebens in die Längsschlitze 76 bei geschlossenem Spannring 42. Fig. 8 zeigt einen vollständig eingeschobenen Sicherungsstift 80. Die Vorsprünge 86 umfassen den unteren Flansch 88 des Spannhebels 72. Durch die Form und Elastizität der beiden Schenkel 84 werden sie fest an diese angedrückt. Zum zerstörungsfreien Herausschieben des Sicherungsstiftes 80 müssen die freien Enden der Schenkel 84 zusammengedrückt werden. Der befugte Anwender verwendet hierzu ein Werkzeug. Der unbefugte Benutzer wird dagegen am Handgriff 90 ziehen. Dabei wird er diesen entlang der Sollbruchstelle 92 vom Steg 82 abtrennen und hierbei ein wenig in Richtung des eingezeichneten Pfeiles bewegen. Die beiden Schenkel 84 mit den Vorsprüngen 86 verbleiben jedoch im Spannring 42 und halten diesen geschlossen. Der befugte Anwender erkennt dann am abgerissenen Handgriff 90, daß ein Unbefugter versucht hat, den Spannring 42 zu öffnen.

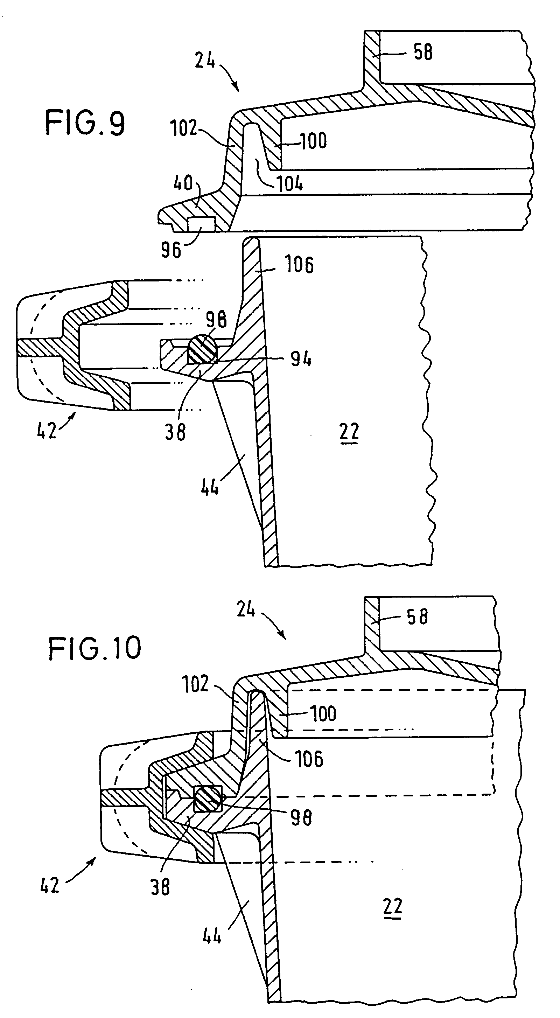

Die Figuren 9 und 10 zeigen die beiden Ringnuten 94 und 96 im Umfangsflansch 38 des Kübels 22 und im Umfangsflansch 40 des Deckels 24. Sie dienen zur Aufnahme eines O-Ringes 98. Die Figuren zeigen weiter einen inneren Ringsteg 100 und einen äußeren Ringsteg 102. Sie umschließen einen Ringspalt 104. Dieser schiebt sich beim Auflegen des Deckels 24 auf den Kübel 22 auf dessen oberes Ende 106. Der Ringspalt 104 hat eine von unten nach oben abnehmende Breite und ein abgerundetes oberes Ende. Fig. 9 zeigt die Lage beim Auflegen des Deckels 24. Fig. 10 zeigt die Lage bei aufgelegtem Deckel 24 und auf die Umfangsflansche 38 und 40 aufgelegtem Spannring 42.

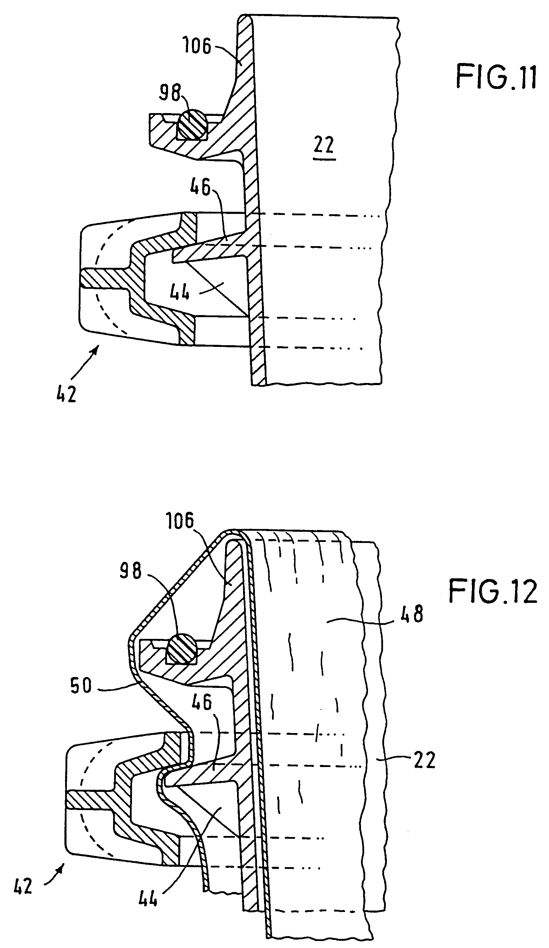

Fig. 11 zeigt den bei Nichtgebrauch auf den Vorsprüngen 46 aufliegenden Spannring 42. Fig. 12 zeigt zusätzlich den eingelegten Innenbeutel 48. Mit seinem oberen Bereich 50 ist er an der Außenwand des Kübels 22 nach unten gezogen. Er liegt sowohl über dem Umfangsflansch 38 als auch über den Vorsprüngen 46. In der Praxis ist nicht erforderlich, daß der Innenbeutel 48 so weit heruntergezogen wird.

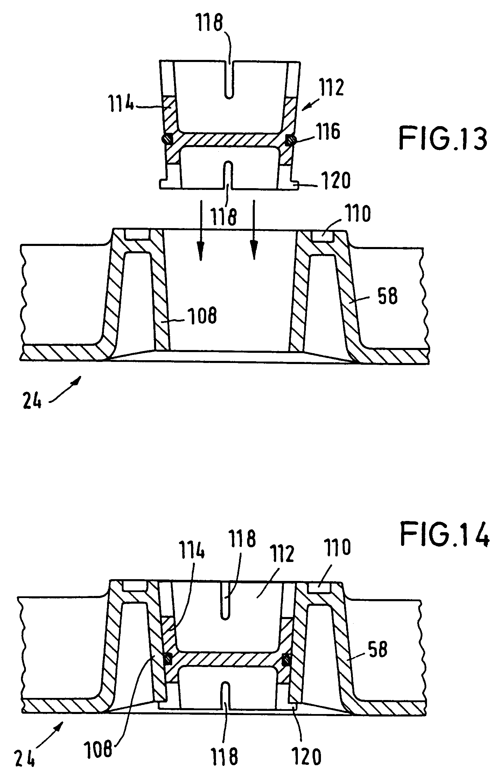

Die Figuren 13 und 14 zeigen eine Alternative zu der in Fig. 4 im Kreis XIII gezeigten Anordnung einer Scheibe 66. Im Rohrstutzen 58 des Deckels 24 ist ein konisch zulaufendes Rohrstück 108 angeordnet. Eine Ringnut 110 liegt zwischen dem Rohrstutzen 58 und dem Rohrstück 108. Beim Einstechen der Injektionsnadel dient sie zur Zentrierung. Die Figuren 13 und 14 zeigen weiter einen Stopfen 112 mit einer konisch zulaufenden Wand 114, einem in seinem Umfang gehaltenen O-Ring 116 und je vier oben und unten in Umfangsrichtung in ihm verteilt angeordneten Schlitzen 118. Am unteren Ende des Stopfens 112 ist noch ein Flansch 120 vorgesehen. Bei vollständig eingeschobenem Stopfen 112 untergreift er das Rohrstück 108.

Im praktischen Betrieb eines Krankenhauses wird der Sterilisationsbehälter mit abgenommenem Deckel 24 aufgestellt. Er nimmt in etwa die aus Fig. 12 ersichtliche Lage ein. Er wird dann mit Abfällen gefüllt. Der gefüllte Sterilisationsbehälter wird mit dem Deckel 24 verschlossen, und der Spannring 42 wird aufgelegt. Die Figuren 9 und 10 zeigen diese Vorgänge. Der Spannring 42 wird geschlossen. Hierzu wird der Spannhebel 72 in Richtung des in Fig. 6 eingezeichneten Pfeiles geschwenkt. Als nächstes wird der Sicherungsstift 80 eingeschoben. Die Figuren 7 und 8 erläutern diese Vorgänge. Die in dem Sterilisationsbehälter enthaltenen Abfälle sind nun gegenüber der Umgebung hermetisch abgedichtet. Zum Sterilisieren der Abfälle werden mehrere gefüllte Sterilisationsbehälter in die hierzu vorgesehene Einrichtung eingestellt. In dieser befinden sie sich an genau vorgegebenen Stellen zentriert unter Injektionsnadeln. Diese werden nach unten geschoben. Dabei durchstoßen sie entweder die in Fig. 4 eingezeichnete Scheibe 66 oder drücken diese sogar nach unten, oder sie durchstoßen die Querwand des in den Figuren 13 und 14 gezeigten Stopfens 112. Über die Injektionsnadeln wird eine Flüssigkeit, zum Beispiel Wasser, in die Sterilisationsbehälter eingespritzt. Die Einstichstelle wird auf eine nicht zur Erfindung gehörende Weise abgedichtet. Anschließend wird die Mikrowellenheizung eingeschaltet und die Abfälle mit Mikrowellen beaufschlagt. Dadurch werden diese erhitzt und sterilisiert. Durch die Entwicklung von Dampf steigt der Druck im Sterilisationsbehälter, und dieser wird geringfügig aufgebläht. Durch die Formgebung von Deckel 24 und Boden 30 und die an mehreren Stellen vorgesehenen Rippen verformt sich der Sterilisationsbehälter jedoch nur so wenig, daß seine Abdichtung gegenüber der Umwelt wie auch seine Unversehrtheit erhalten bleiben. Nach Abschluß der Erhitzung und der Sterilisation wird der Sterilisationsbehälter zur Entsorgung bereitgestellt. Dabei kühlt er sich ab. In seinem Inneren kann ein gewisser Unterdruck entstehen. Auch hier sorgen die verschiedenen Rippen für Dichtigkeit und Unversehrtheit des Sterilisationsbehälters.

Nach Entsorgen der sterilisierten Abfälle und gegebenenfalls nach einem Reinigen der Kübel 22 und der Deckel 24 werden diese erneut verwendet. Hierzu können die Kübel 22, wie in Fig. 1 angedeutet, gestapelt werden. Auch die Deckel 24 können übereinandergestapelt werden.

## Patentansprüche

1. Sterilisationsbehälter, insbesondere für Krankenhausabfälle, mit einem Kübel und einem auf diesen aufsetzbaren Deckel aus einem zähen, für Mikrowellen durchlässigen und auch über die Siedetemperatur von Wasser hinaus dehnungsfesten Kunststoff und mit einer Abdichtung zwischen dem oberen Rand des Kübels und dem aufgelegten Deckel, gekennzeichnet durch eine abnehmbare Verschlußanordnung zum Halten des Deckels (24) auf dem Kübel (22) entgegen dem Druck eines im Sterilisationsbehälter zur Sterilisation erzeugten Dampfes.

2. Sterilisationsbehälter nach Anspruch 1, dadurch gekennzeichnet, daß der Kübel (22) aus mehreren sich in ihrem Durchmesser von oben nach unten verjüngenden Abschnitten (26) besteht.

3. Sterilisationsbehälter nach Anspruch 1 oder 2, gekennzeichnet durch in Umfangsrichtung verteilt angeordnete, von der Innenseite des Bodens (30) eines Kübels (22) an dessen Innenwand nach oben verlaufende und in ihrer radialen Stärke abnehmende Rippen (32) zur Auflage eines in diesen Kübel (22) eingesetzten weiteren Kübels (22).

4. Sterilisationsbehälter nach einem der Ansprüche 1 bis 3, gekennzeichnet durch einen nach oben gewölbten Boden (30) und einen auf der Unterseite des Bodens (30) eines Kübels (22) mittig angeordneten Rohrstutzen (34), dessen Höhe unter der Auswölbung des Bodens (30) liegt.

5. Sterilisationsbehälter nach Anspruch 4, gekennzeichnet durch in Umfangsrichtung verteilt vom Umfang des Rohrstutzens (34) zur Unterseite des Bodens (30) eines Kübels (22) verlaufende und in ihrer Höhe abnehmende Rippen (36).

6. Sterilisationsbehälter nach einem der Ansprüche 1 bis 5, dadurch gekennzeichnet, daß der Deckel (24) nach unten gewölbt ist.

7. Sterilisationsbehälter nach einem der Ansprüche 1 bis 6, gekennzeichnet durch einen auf der Oberseite des Deckels (24) mittig angeordneten Rohrstutzen (58).

8. Sterilisationsbehälter nach einem der Ansprüche 1 bis 7, gekennzeichnet durch einen auf der Innenseite des Deckels (24) nahe an dessen Umfang umlaufenden und bei ineinandergesetzten Kübeln (22) an der Innenwand eines Kübels (22) anliegenden inneren Ringsteg (100).

9. Sterilisationsbehälter nach einem der Ansprüche 1 bis 8, gekennzeichnet durch in Umfangsrichtung verteilt vom Rohrstutzen (58) auf der Oberseite des Deckels (24) zu dessen Umfang verlaufende und in ihrer Höhe abnehmende Rippen (56).

10. Sterilisationsbehälter nach einem der Ansprüche 1 bis 9, gekennzeichnet durch in Umfangsrichtung verteilt vom Umfang des Deckels (24) auf dessen Untereite zur Mitte hin verlaufende und in ihrer Höhe abnehmende Rippen (60).

11. Sterilisationsbehälter nach einem der Ansprüche 1 bis 10, gekennzeichnet durch ein im Rohrstutzen (58) des Deckels (24) angeordnetes konisch verlaufendes Rohrstück (108) mit von oben nach unten abnehmendem Durchmesser und einem in dieses hineindruckbaren Stopfen (112) aus einem elastischen Werkstoff.

12. Sterilisationsbehälter nach Anspruch 11, dadurch gekennzeichnet, daß der Stopfen (112) hohl ist.

13. Sterilisationsbehälter nach Anspruch 11 und 12, gekennzeichnet durch im Mantel des Stopfens (112) vorgesehene axial verlaufende Schlitze (118) und einen am unteren Ende vorgesehenen, das Rohrstück (108) untergreifenden Flansch (120).

14. Sterilisationsbehälter nach Anspruch 11 bis 13, gekennzeichnet durch einen auf dem Mantel des Stopfens (112) gehaltenen O-Ring (116).

15. Sterilisationsbehälter nach einem der Ansprüche 1 bis 14, dadurch gekennzeichnet, daß ein Umfangsflansch (38) am oberen Rand (106) des Kübels (22) und ein dazu passender Umfangsflansch (40) am Rand des Deckels (24) vorgesehen ist, eine Nut (94, 96) in diesen ausgebildet und ein 0-Ring (98) in dieser Nut aufgenommen ist.

16. Sterilisationsbehälter nach einem der Ansprüche 1 bis 15, dadurch gekennzeichnet, daß die Abmessungen der Nut (94, 96) so auf die Abmessungen des O-Ringes (98) abgestimmt sind, daß dieser im Betriebszustand zusammengepreßt ist.

17. Sterilisationsbehälter nach einem der Ansprüche 1 bis 16, dadurch gekennzeichnet, daß die Verschlußanordnung ein Spannring (42) ist und dieser auf die Umfangsflansche (38, 40) von Kübel (22) und Deckel (24) auflegbar ist.

18. Sterilisationsbehälter nach Anspruch 17, dadurch gekennzeichnet, daß der Spannhebel (72) des Spannringes (42) in Schließstellung durch einen in ihn und den Spannring (43) einschiebbaren Sicherungsstift (80) verriegelbar ist.

19. Sterilisationsbehälter nach Anspruch 18, dadurch gekennzeichnet, daß der Spannring (42) und der Spannhebel (72) in Schließstellung übereinanderliegende Öffnungen aufweisen und der Sicherungsstift (80) in diese einschiebbar ist.

20. Sterilisationsbehälter nach Anspruch 19, dadurch gekennzeichnet, daß die Öffnungen Längsschlitze (76) sind.

21. Sterilisationsbehälter nach einem der Ansprüche 17 bis 20, dadurch gekennzeichnet, daß eine radial vorspringende Lasche (74) an den Spannring (42) angesetzt und der Längsschlitz (76) in dieser ausgebildet ist.

22. Sterilisationsbehälter nach einem der Ansprüche 17 bis 21, dadurch gekennzeichnet, daß der Sicherungsstift (80) zwei an ihren oberen Enden durch einen Steg (82) verbundene Schenkel (84) aufweist und ein Handgriff (90) über eine Sollbruchstelle (92) mit dem Steg (82) verbunden ist.

23. Sterilisationsbehälter nach Anspruch 22, dadurch gekennzeichnet, daß der Handgriff (90) die Form eines Ringes aufweist.

24. Sterilisationsbehälter nach einem der Ansprüche 17 bis 23, dadurch gekenneichnet, daß die beiden Schenkel (84) eine mit einer Begrenzung eines Längsschlitzes (76) verrastbare Sicherung aufweisen.

25. Sterilisationsbehälter nach Anspruch 24, dadurch gekennzeichnet, daß die Sicherung aus sich von beiden Seiten an die Begrenzung des Längsschlitzes (76) anlegenden Vorsprüngen (86) der Schenkel (84) besteht.

26. Sterilisationsbehälter nach Anspruch 25, dadurch gekennzeichnet, daß die Vorsprünge (86) an den freien unteren Enden der Schenkel (84) angeordnet sind.

27. Sterilisationsbehalter nach einem der Ansprüche 17 bis 26, dadurch gekennzeichnet, daß die freien unteren Enden der Schenkel (84) elastisch zusammendrückbar sind.

28. Sterilisationsbehälter nach einem der Ansprüche 1 bis 27, gekennzeichnet durch auf der Außenwand eines Kübels (22) in einem Abstand unter dessen oberem Rand (106) in Umfangsrichtung verteilt angeordnete radiale Vorsprünge (46), wobei der Abstand zum oberen Rand (106) kleiner als die Höhe der Rippen (32) ist und auf der Außenwand des Kübels (22) bis zu den Unterseiten der Vorsprünge (46) unter radialem Abstand verlaufende Rippen (44).

## Claims

1. A sterilisation container, in particular for hospital waste, having a bin and a lid which can be fitted on to the bin and which comprises a tough plastics material which is microwave-transparent and which is stretch-resistant even beyond the boiling temperature of water, and having a seal between the upper edge of the bin and the fitted lid, characterised by a removable closure arrangement for holding the lid (24) on the bin (22) against the pressure of a steam generated in the sterilisation container for sterilisation purposes.

2. A sterilisation container according to claim 1 characterised in that the bin (22) comprises a plurality of portions (26) which narrow in their diameter in a downward direction.

3. A sterilisation container according to claim 1 or claim 2 characterised by ribs (32) which are arranged distributed in the peripheral direction and which extend upwardly on the inside wall of a bin (22) from the inside of the bottom (30) thereof and which decrease in their radial dimension, for supporting a further bin (22) which is fitted into said bin (22).

4. A sterilisation container according to one of claims 1 to 3 characterised by an upwardly curved bottom (30) and a tubular socket portion (34) which is arranged centrally on the underside of the bottom (30) of a bin (22) and whose height is below the curvature of the bottom (30).

5. A sterilisation container according to claim 4 characterised by ribs (36) which extend distributed in the peripheral direction from the periphery of the tubular socket portion (34) to the underside of the bottom (30) of a bin (22) and which decrease in height.

6. A sterilisation container according to one of claims 1 to 5 characterised in that the lid (24) is curved downwardly.

7. A sterilisation container according to one of claims 1 to 6 characterised by a tubular socket portion (58) arranged centrally on the top side of the lid (24).

8. A sterilisation container according to one of claims 1 to 7 characterised by an inner annular flange (100) which extends around the lid (24) on the inside thereof in the proximity of the periphery thereof and which when bins (22) are fitted one into the other bears against the inside wall of a bin (22).

9. A sterilisation container according to one of claims 1 to 8 characterised by ribs (56) which extend distributed in the peripheral direction from the tubular socket portion (58) on the top side of the lid (24) to the periphery thereof and which decrease in height.

10. A sterilisation container according to one of claims 1 to 9 characterised by ribs (60) which extend distributed in the peripheral direction from the periphery of the lid (24) on the underside thereof towards the centre and which decrease in height.

11. A sterilisation container according to one of claims 1 to 10 characterised by a conically extending tubular portion (108) with a diameter which decreases in a downward direction, the tubular portion (108) being arranged in the tubular socket portion (58) of the lid (24), and a plug (112) of an elastic material which can be pressed into said tubular portion.

12. A sterilisation container according to claim 11 characterised in that the plug (112) is hollow.

13. A sterilisation container according to claim 11 and claim 12 characterised by axially extending slots (118) provided in the peripheral portion of the plug (112), and a flange (120) which is provided at the lower end and which engages under the tubular portion (108).

14. A sterilisation container according to claims 11 to 13 characterised by an O-ring (116) carried on the peripheral portion of the plug (112).

15. A sterilisation container according to one of claims 1 to 14 characterised in that a peripheral flange (38) is provided at the upper edge (106) of the bin (22) and a peripheral flange (40) fitting thereto is provided at the edge of the lid (24), a groove (94, 96) is provided in said peripheral flanges and an O-ring (98) is accommodated in said groove.

16. A sterilisation container according to one of claims 1 to 15 characterised in that the dimensions of the groove (94, 96) are so matched to the dimensions of the O-ring (98) that it is compressed in the operative condition.

17. A sterilisation container according to one of claims 1 to 16 characterised in that the closure arrangement is a clamping ring (42) and it can be fitted on to the peripheral flanges (38, 40) of the bin (22) and the lid (24).

18. A sterilisation container according to claim 17 characterised in that the clamping lever (72) of the clamping ring (42) is lockable in the closure position by a securing pin (80) which can be inserted into the clamping lever (72) and the clamping ring (42).

19. A sterilisation container according to claim 18 characterised in that the clamping ring (42) and the clamping lever (72) have openings which are in mutually superposed relationship in the closure position and the securing pin (80) is insertable into the openings.

20. A sterilisation container according to claim 19 characterised in that the openings are longitudinal slots (76).

21. A sterilisation container according to one of claims 17 to 20 characterised in that a radially projecting tongue (74) is fitted to the clamping ring (42) and the longitudinal slot (76) is provided in the tongue.

22. A sterilisation container according to one of claims 17 to 21 characterised in that the securing pin (80) has two limbs (84) which are connected at their upper ends by a web (82) and a handle (90) is connected to the web (82) by way of a desired-rupture location (92).

23. A sterilisation container according to claim 22 characterised in that the handle (90) is in the form of a ring.

24. A sterilisation container according to one of claims 17 to 23 characterised in that the two limbs (84) have a securing means which is latchable to a boundary of a longitudinal slot (76).

25. A sterilisation container according to claim 24 characterised in that the securing means comprises projections (86) on the limbs (84), which projections bear against the boundary of the longitudinal slot (76) from both sides.

26. A sterilisation container according to claim 25 characterised in that the projections (86) are arranged at the free lower ends of the limbs (84).

27. A sterilisation container according to one of claims 17 to 26 characterised in that the free lower ends of the limbs (84) can be elastically pressed together.

28. A sterilisation container according to one of claims 1 to 27 characterised by radial projections (46) which are arranged distributed in the peripheral direction on the outside wall of a bin (22) at a spacing below the upper edge (106) thereof, wherein the spacing relative to the upper edge (106) is smaller than the height of the ribs (32), and ribs (44) extending on the outside wall of the bin (22) to the undersides of the projections (46) at a radial spacing.

## Revendications

1. Récipient de stérilisation, en particulier pour déchets d'hôpital, avec un seau et avec un couvercle en une matière synthétique tenace, perméable aux mirco-ondes et résistante à la dilatation même au-delà de la température d'ébullition d'eau, qui peut être posé sur ledit seau, et avec un moyen d'étanchéité entre le bord supérieur du seau et le couvercle posé, caractérisé par une disposition de fermeture amovible destinée à tenir le couvercle (24) sur le seau (22) contre la pression d'une vapeur génerée dans le récipient de stérilisation pour la stérilisation.

2. Récipient de stérilisation selon la revendication 1, caractérisé par le fait que le seau (22) se compose de plusieurs segments (26) dont le diamètre se rétrécit de haut en bas.

3. Récipient de stérilisation selon la revendication 1 ou 2, caractérisé par des nervures (32) réparties dans la direction circonférentielle, s'étendant de la face interne du fond (30) d'un seau (22) sur sa paroi intérieure vers le haut et dont l'épaisseur radiale diminue, qui sont destinées à appuyer un autre seau (22) inséré dans ce seau (22).

4. Récipient de stérilisation selon l'une des revendications 1 à 3, caractérisé par un fond (30) bombé vers le haut et par une tubulure (34) disposée de façon centrale sur la face inférieure du fond (30) d'un seau (22), dont la hauteur est inférieure au bombement du fond (30).

5. Récipient de stérilisation selon la revendication 4, caractérisé par des nervures (36) réparties dans la direction circonférentielle, s'étendant de la circonférence de la tubulure (34) vers la face inférieure du fond (30) d'un seau (22) et présentant une hauteur qui diminue.

6. Récipient de stérilistation selon l'une des revendications 1 à 5, caractérisé par le fait que le couvercle (24) est bombé vers le bas.

7. Récipient de stérilisation selon l'une des revendications 1 à 6, caractérisé par une tubulure (58) disposée de façon centrale sur la surface supérieure du couvercle (24).

8. Récipient de stérilisation selon l'une des revendications 1 a 7, caractérisé par une entretoise annulaire intérieure (100) s'étendant tout autour sur la face interne du couvercle (24) prés de la périphérie de celui-ci et s'appuyant sur la paroi intérieure d'un seau (22), lorsque des seaux (22) sont insérés l'un dans l'autre.

9. Récipient de stérilisation selon l'une des revendications 1 à 8, caractérisé par des nervures (56) réparties dans la direction circonférentielle, s'étendant de la tubulure (58) sur la surface supérieure du couvercle (24) vers la circonférence de celui-ci et présentant une hauteur qui diminue.

10. Récipient de stérilisation selon l'une des revendications 1 à 9, caractérisé par des nervures (60) réparties dans la direction circonférentielle, s'étendant de la circonférence du couvercle (24) sur la face inférieure de celui-ci vers le milieu et présentant une hauteur qui diminue.

11. Récipient de stérilisation selon l'une des revendications 1 à 10, caractérisé par une partie de tuyau (108) disposée dans la tubulure (58) du couvercle (24) et s étendant de façon conique, qui présente un diamètre diminuant de haut en bas ainsi qu'un bouchon (112) en un matériau élastique qui peut être fourré dans ladite partie de tuyau.

12. Récipient de stérilisation selon la revendication 11, caractérisé par le fait que le bouchon (112) est creux.

13. Récipient de stérilisation selon la revendication 11 et 12, caractérisé par des fentes (118) prévues dans la nappe du bouchon (112) et s'étendant axialement, et par une bride (120) prévue à l'extrémité inférieure, qui se prend sous la partie de tuyau (108).

14. Récipient de stérilisation selon la revendication 11 à 13, caractérisé par un O-ring (116) tenu sur la nappe du bouchon (112).

15. Récipient de stérilisation selon l'une des revendications 1 à 14, caractérisé par le fait que l'on prévoit une bride circonférentielle (38) sur le bord supérieur (106) du seau (22) et une bride circonférentielle (40) sur le bord du couvercle (24), qui s ajuste à cette première, qu'une rainure (94, 96) est formée dans celles-ci et qu'un O-ring (98) est reçu dans cette rainure.

16. Récipient de stérilisation selon l'une des revendications 1 a 15, caractérisé par le fait que les dimensions de la rainure (94, 96) sont accordées aux dimensions du O-ring (98) de telle manière que ce dernier est comprimé en état de fonctionnement.

17. Récipient de stérilisation selon l'une des revendications 1 à 16, caractérisé par le fait que la disposition de fermeture est un anneau de serrage (42) et que celui-ci peut être posé sur les brides circonférentielles (38, 40) du seau (22) et du couvercle (24).

18. Récipient de stérilisation selon la revendication 17, caractérisé par le fait que le levier de serrage (72) de l'anneau de serrage (42) peut être verrouillé, en position fermée, par le bais d'une goupille de sécurité (80) qui peut être introduite en glissant dans ce premier et dans l'anneau de serrage (42).

19. Récipient de stérilisation selon la revendication 18, caractérisé par le fait que, en position fermée, l'anneau de serrage (42) et le levier de serrage (72) présentent des ouvertures situées l'une sur l'autre, et que la goupille de sécurité (80) peut être introduite en glissant dans celles-ci.

20. Récipient de stérilisation selon la revendication 19, caractérisé par le fait que les ouvertures sont des fentes longitudinales (76).

21. Récipient de stérilisation selon l'une des revendications 17 à 20, caractérisé par le fait qu'une languette (74) saillant radialement est rapportée a l'anneau de serrage (42) et que la fente longitudinale (76) est réalisée dans celle-ci.

22. Récipient de stérilisation selon l'une des revendications 17 à 21, caractérisé par le fait que la goupille de sécurité (80) présente deux branches (84) qui, à leur extrémité supérieure, sont reliées entre elles par l'intermédiaire d'une entretoise (82) et qu'une poignée (90) est reliée par un point destiné à la rupture (92) a l'entretoise (82).

23. Récipient de stérilisation selon la revendication 22, caractérisé par le fait que la poignée (90) présente la forme d'un anneau.

24. Récipient de stérilisation selon l'une des revendications 17 a 23, caractérisé par le fait que les deux branches (84) présentent un dispositif de sécurité qui peut être enclenché avec une limitation d'une fente longitudinale (76).

25. Récipient de stérilisation selon la revendication 24, caractérisé par le fait que le dispositif de sécurité se compose de projections (86) des branches (84), qui s'appuient des deux côtés sur la limitation de la fente longitudinale (76).

26. Récipient de stérilisation selon la revendication 25, caractérisé par le fait que les projections (86) sont disposées aux extrémités libres inférieures des branches (84).

27. Récipient de stérilisation selon l'une des revendications 17 a 26, caractérisé par le fait que les extrémités libres inférieures des branches (84) peuvent être pressées l'une contre l'autre de manière élastique.

28. Récipient de stérilisation selon l'une des revendications 1 a 27, caractérisé par des projections radiales (46) réparties sur la paroi extérieure d'un seau (22) à une distance au-dessous du bord supérieur (106) de celui-ci dans la direction circonférentielle, la distance au bord supérieur (106) étant plus petite que la hauteur des nervures (32), et par des nervures (44) présentant une distance radiale l'une de l'autre qui s'étendent sur la paroi extérieure du seau (22) jusqu'aux faces inférieures des projections (46).
